(19) Europäisches Patentamt / European Patent Office / Office européen des brevets

(11) **EP 3 033 076 B1**

(12) **EUROPEAN PATENT SPECIFICATION**

(45) Date of publication and mention
of the grant of the patent:
**25.11.2020 Bulletin 2020/48**

(51) Int Cl.:
***A61K 9/28*** *(2006.01)*     ***A61K 31/40*** *(2006.01)*
***A61K 47/32*** *(2006.01)*

(21) Application number: **14745154.6**

(22) Date of filing: **01.08.2014**

(86) International application number:
**PCT/EP2014/066601**

(87) International publication number:
**WO 2015/022204 (19.02.2015 Gazette 2015/07)**

(54) **COATING COMPOSITION**

BESCHICHTUNGSZUSAMMENSETZUNG

COMPOSITION DE REVÊTEMENT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **14.08.2013 IN CH36232013**

(43) Date of publication of application:
**22.06.2016 Bulletin 2016/25**

(73) Proprietor: **Evonik Operations GmbH
45128 Essen (DE)**

(72) Inventors:
• **JOSHI, Shraddha Sanjeev
Navi Mumbai
Maharashtra 400709 (IN)**
• **JAIN, Vinay
Indore
Madhya Pradesh 452003 (IN)**
• **GUHA, Ashish Sharadchandra
Dombivali
Mumbai 421204 (IN)**

(74) Representative: **Evonik Patent Association
c/o Evonik Industries AG
IP Management
PB 84/339
Rodenbacher Chaussee 4
63457 Hanau (DE)**

(56) References cited:
**WO-A1-2004/052345    US-A1- 2009 175 937
US-A1- 2011 281 855    US-A1- 2011 305 756**

**Description**

**Technical field**

The invention discloses a coating composition which, calculated on dry weight, is comprising at least 20 % by weight polyvinyl alcohol and 0.1 to 20 % by weight, calculated on the weight of the polyvinyl alcohol, of a water-insoluble (meth)acrylate copolymer comprising quaternary ammonium groups, wherein the viscosity of an aqueous dispersion comprising that coating composition with 25 % (w/w) solid content at 22 °C is 200 mPa·s or less measured at 22 °C using a Brookfield Viscometer.

**Technical background**

WO 2010/132204A1 describes an immediate release film coating composition with enhanced moisture barrier properties comprising polyvinyl alcohol, a polymer with pH dependent solubility, and optionally a plasticizer and/or a glidant. The polymer with pH dependent solubility may be for instance hydroxypropylmethyl cellulose acetate succinate (HPMC-AS) or EUDRAGIT® L100-55.

**Object of the invention**

WO 2010/132204A1 describes an immediate release film coating composition with enhanced moisture barrier properties. However it was found that the viscosity of the polymer composition is comparatively high what is considered to be of disadvantage in spray coating applications. Furthermore it was found that the presence of a polymer with pH dependent solubility in the coating of a coated core may negatively influence the stability of acid labile compounds in the core due its acidic nature. Thus it was an object of the present invention when starting from WO 2010/132204A1 to provide an alternative composition with improved viscosity parameters and improved conditions that may influence the stability of active ingredients included.
The problem was solved as claimed.

**Details of the invention**

**Coating composition**

The invention refers to a coating composition which, calculated on dry weight, is comprising at least 20 % by weight polyvinyl alcohol and 0.1 to 20 % by weight, calculated on the weight of the polyvinyl alcohol, of a water-insoluble (meth)acrylate copolymer comprising quaternary ammonium groups, wherein the viscosity of an aqueous dispersion comprising that coating composition with 25 % (w/w) solid content at 22 °C is 200 mPa·s or less measured at 22 °C using a Brookfield Viscometer.
The invention refers to a coating composition which, calculated on dry weight, may comprise at least 20, at least 30, at least 40, at least 50, at least 60, at least 70, at least 80 % by weight polyvinyl alcohol. The coating composition may comprise 20 to 80, 30 to 70, 40 to 60 % by weight polyvinyl alcohol.
The coating composition may comprise, calculated on dry weight, 0.1 to 20, 0.2 to 10, 0.5 to 5 % by weight, calculated on the weight of the polyvinyl alcohol, of a water-insoluble (meth)acrylate copolymer comprising quaternary ammonium groups.
The coating composition may have the form of a powder, for instance with a particle size or a particle size distribution of less than 1000, less than 850, less than 150 μm, preferably in the range of 5 - 1000, 5 to less than 1000, 10 - 850 , 10 to less than 850, 10 - 150 or 150 to less than 150 μm. The particle size or particle size distribution of the powder components may be preferably determined by particle size distribution estimation by analytical sieving according to United States Pharmacopeia (USP), preferably USP 29, method <786>. Another highly suitable measuring method is laser defraction for determination of particle size distribution. Commercial instruments permit measurement in air (Malvern Co. S3.01 Particle Sizer) or preferably in liquid media (LOT Co., Galai CIS 1).
The powder may be dispersed in water to give an aqueous dispersion.
The coating composition may have the form of an aqueous dispersion, for instance with a solid content of 5 - 60, 10 - 40 or 15 or 40 % (weight/volume).
Polyvinyl alcohol and the water-insoluble (meth)acrylate copolymer comprising quaternary ammonium groups may add up to 100% (dry weight). However as a rule the coating composition may also comprise further excipients. In this case polyvinyl alcohol, the water-insoluble (meth)acrylate copolymer comprising quaternary ammonium groups and the further excipients may add up to 100% (dry weight).

## Disintegration

A tablet, for instance a tablet with a diameter of 5 - 15 mm and a coating of 2 to 8 % based on the tablet core, coated with an aqueous dispersion comprising the inventive coating composition disintegrates in an aqueous medium in 30 min or less, 20 min or less, 10 min or less or within 1 to 30 min.

## Further excipients

The coating composition may also comprise further excipients are selected from the group of antioxidants, brighteners, binding agents, flavouring agents, flow aids, fragrances, glidants, penetration-promoting agents, pigments, plasticizers, polymers, which are not a polyvinyl alcohol or a water-insoluble (meth)acrylate copolymer, pore-forming agents or stabilizers.

## Disintegration

A tablet coated with an aqueous dispersion comprising the coating composition disintegrates in an aqueous medium in 30 min or less, in 20 min or less, in 15 min or less or within 1 to 30 min.

## Viscosity

The viscosity of an aqueous dispersion with 25 % (w/w) solid content at 22 °C is 200 mPa.s or less, 180 mPas or less, 150 mPas or less, 120 mPas or less, or 100 mPas or less. The viscosity may be preferably measured at 22 °C using Brookfield Viscometer. A 500 ml glass beaker and a spindle S61 may be used in the study and the determination may be carried out at 20 RPM.

## Water-insoluble (meth)acrylate copolymer comprising quaternary ammonium groups

Water-insoluble (meth)acrylate copolymers in the sense of the invention are polymers which do not dissolve in water or are only swellable in water over of the whole range of pH 1 - 14.

The water-insoluble (meth)acrylate polymer may be composed of 85 to 98% by weight of free-radical polymerized $C_1$- to $C_4$-alkyl esters of acrylic or methacrylic acid and 2 to 15 % by weight of alkyl(meth)acrylate monomers with a quaternary amino group in the alkyl radical.

The water-insoluble (meth)acrylate copolymer may be composed of 50 to 70% by weight of methyl methacrylate, 20 to 40% by weight of ethyl acrylate and more than 7 and up to 15 % by weight of 2-trimethylammoniumethyl methacrylate chloride (type EUDRAGIT® RL).

The water-insoluble (meth)acrylate copolymer may be composed of 55 to 75 % by weight of methyl methacrylate, 20 - 40% by weight of ethyl acrylate and 2 to 7% by weight of 2-trimethylammoniumethyl methacrylate chloride (type EUDRAGIT® RS).

## Active pharmaceutical or nutraceutical Ingredients

The embodiments of present invention are useful for all kinds of active pharmaceutical or nutraceutical ingredients. Preferably the embodiments of present invention are useful for acid labile active pharmaceutical or nutraceutical ingredients. An acid labile pharmaceutical or nutraceutical ingredient in the sense of the invention may be characterized in that it produces impurities by degradation due to presence of acidic components. An example for an acid labile pharmaceutical ingredient is atorvastatin. The acidic compound under which influence an acid labile pharmaceutical or nutraceutical ingredient produces impurities by degradation may be an acidic substance in the coating. An acidic substance in the coating may be an acid which is added to the coating or a film coating polymer with acidic or carboxylic groups.

Active nutraceutical ingredients or nutraceuticals in general can be defined as extracts of foods claimed to have medical effects on human health. The nutraceutical is usual contained in a medical format such as capsule, tablet or powder in a prescribed dose. Examples for nutraceuticals are resveratrol from grape products as an antioxidant, soluble dietary fiber products, such as psyllium seed husk for reducing hypercholesterolemia, broccoli (sulphane) as a cancer preservative, and soy or clover (isoflavonoids) to improve arterial health. Other nutraceuticals examples are flavonoids, antioxidants, alpha-linoleic acid from flax seed, beta-carotene from marigold petals or antocyanins from berries. Sometimes the expression neutraceuticals is used as synonym for nutraceuticals.

### Dosage form

The invention discloses a dosage form, preferably an orally ingestible dosage form, comprising a core, comprising an active pharmaceutical or nutraceutical ingredient and a coating derived from a coating composition as described herein.

The dosage form may preferably comprise an active pharmaceutical or nutraceutical ingredient is acid labile.

### Atorvastatin pellets

The coating composition may be characterized in that, when used for coating of atorvastatin containing pellets, the increase in impurities measured as lactone from the initial stage (day 0) to 45 days storage in open petri dishes at 40 °C and 75 % relative humidity is less than 1000, less than 500, less than 250, less than 200, less than 150 or less than 100 %.

The (relative) increase of impurities is calulated by subtracting the initial value of impurities from the coated atorvastatin containing pellets from the value impurities of the coated atorvastatin containing pellets after 45 days storage in open petri dishes at 40 °C and 75 % relative humidity and calculating the percentage of the this difference in comparison to the initial value (100%).

The lactone impurities of the coated formulations may be measured by HPLC or other suitable analytical methods.

### Use

The invention discloses the use of a coating composition as described herein for preparing a dosage form as also described herein.

### Process

The invention discloses a process for preparing a dosage form as described herein by first preparing a core comprising an active pharmaceutical or nutraceutical ingredient and subsequently applying a coating composition as described herein onto the core.

### Examples

Examples with a "C" are comparative examples. Examples without a "C" are inventive examples.

### Materials and Methods

PVA: Polyvinyl alcohol

Poval® 205S: is a commercially available product comprising polyvinyl alcohol.

EUDRAGIT® L 100-55 is a copolymer composed of polymerized units of 50% by weight ethyl acrylate and 50% by weight methacrylic acid.

EUDRAGIT® RL 30D is a 30 % (dry substance) aqueous dispersion of a copolymer composed of polymerized units of 60% by weight of methyl methacrylate, 30% by weight of ethyl acrylate and 10% by weight of 2-trimethylammoniumethyl methacrylate chloride.

EUDRAGIT® NE 30D and Eudragit® NM 30D are 30 % (dry substance) aqueous dispersions of copolymers composed of polymerized units of 30% by weight of ethyl acrylate and 70% by weight of methyl methacrylate.

Opadry® 200 is a commercially available product comprising polyvinyl alcohol, EUDRAGIT L 100-55, sodium bicarbonate, talc, titanium dioxide and/or colour.

Opadry® AMB is a commercially available product comprising polyvinyl alcohol, lecithin, talc, titanium dioxide and/or colour.

Parts A and B:

Viscosity was measured at 22 °C using Brookfield Viscometer. 500 ml glass beaker and spindle S61 was used in the study and determination was carried out at 20 RPM.

Part C:

The impurities measured as lactone in coated formulations was measured by HPLC.

The increase of impurities is calulated by subtracting the initial value of impurities from the coated atorvastatin containing pellets from the value impurities of the coated atorvastatin containing pellets after 45 days storage in open petri dishes at 40 °C and 75 % relative humidity and calculating the percentage of the this difference in comparison to the initial value (100%).

**RELATED SUBSTANCES (by HPLC):**

**Chromatographic conditions:**

| Column | Zorbax Rx C8, 250 x 4.6mm, 5micron or equivalent |
|---|---|
| Detection | UV-Visible |
| Wavelength | 244nm |
| Flow rate | 1.5 ml/min |
| Injection volume | 20 $\mu$l |
| Run Time | 115 minutes |
| Column Temperature | 35°C |
| Diluent | Water: Acetonitrile (60:40) |

**Mobile phase A:** Mix Tetrahydrofuran, Acetonitrile and ammonium acetate solution in the ratio of (12:21:67).

**Mobile phase B:** Mix Tetrahydrofuran, ammonium acetate solution and Acetonitrile in the ratio of (12:27:61).

**Preparation of Ammonium Acetate solution:** Weigh about 3.9 g of Ammonium Acetate and dissolve in 1000 ml of Water and adjust pH to 5.00 with Acetic Acid.

| Gradient proqram: Time (min) | Mobile phase A (%) | Mobile phase B (%) |
|---|---|---|
| 0-40 | 100 | 0 |
| 40-70 | 100-20 | 0-80 |
| 70-85 | 20-0 | 80-100 |
| 85-100 | 0 | 100 |
| 100-105 | 0-100 | 100-0 |
| 105-115 | 100 | 0 |

**Test solution:** Weigh sample equivalent to 0.050g of atorvastatin and transfer in to 50 ml volumetric flask. Add 30ml diluent and sonicate for 30 min with intermittent shaking. Allow cool to room temperature. Dilute to volume with diluent.

**Reference solution:** Weigh accurately about 0.050g of Atorvastatin calcium working standard in to 50 ml volumetric flask, dissolve and dilute to volume with diluents (stock solution). Dilute 1 ml of stock solution to 100 ml with diluent. Further, dilute 1 ml of this solution to 10 ml with diluent.

| NAME | Relative Retention Time | Response Factor (RF) |
|---|---|---|
| Atorvastatin lactone | about 1.72 | 1.0 |

**Calculation:**

Calculate the % Related substances by using the below calculation formula.

$$\frac{\text{Area of impurity in Test solution} \times \text{wt of standard} \times 1 \times 1 \times 50 \times 100 \times RF \times \text{Average wt}}{\text{Mean area of Reference solution} \times 50 \times 100 \times 10 \times \text{wt of sample} \times \text{Lable claim}}$$

Part D:

Moisture protection of the inventive formulation at 84% relative humidity (RH)

**Part A: Examples C1 and 2: Processing advantage of the inventive formulation**

**Procedure for examples C1 and 2**

25%w/w dispersion were prepared with a PVA (Poval® 205S) + EUDRAGIT® L100-55 formulation (example C1, comparative according to WO 2010/132204A1, Table 1) and PVA+RL combination (example 2, inventive) under overhead stirring for 1 hour. The prepared coating dispersions were filtered through 60# sieve and sprayed onto Placebo tablets using 18" perforated coating pan (Neocota)

Table 1

| Example C1 PVA+EUDRAGIT® L100-55 formulation | |
|---|---|
| **Ingredients** | **% content** |
| Poval® 205S | 36.87 |
| EUDRAGIT® L100-55 | 4.22 |
| PEG 3350 | 12.64 |
| Talc | 25.07 |
| Titanium di oxide | 21.07 |
| Sodium bi Carbonate | 0.13 |
| Total | 100.00 |
| Water | to be added to a Solid content of 25%w/w |

Table 2

| Example 2 PVA+EUDRAGIT® RL formulation | |
|---|---|
| **Ingredients** | **% content** |
| | |
| Poval® 205S | 40.29 |
| EUDRAGIT® RL 30 D(on dry basis) | 0.8 |
| PEG 3350 | 12.64 |
| Talc | 25.2 |
| Titanium di oxide | 21.07 |
| Total | 100.00 |

(continued)

| Example 2<br>PVA+EUDRAGIT® RL formulation | |
|---|---|
| Ingredients | % content |
| Water | to be added to a Solid content of 25%w/w |

**Results:**

Table 3

| | PVA+EUDRAGIT® L100-55 formulation | PVA+EUDRAGIT® RL 30D formulation |
|---|---|---|
| Solid content | 25% w/w | 25% w/w |
| Batch Size | 4 kg Placebo tablets | 4 kg Placebo tablets |
| Spray rate | **Up to 17 gm/min-processible** | **Up to 25 gm/min**-No sticking observed |
| | Above 17gm/min-Tablets were tacky and sticking observed | |
| Viscosity | **260 mPa.s** | **100 mPa.s** |

The inventive formulation of example 2 (PVA+EUDRAGIT® RL formulation) has a lower viscosity and can be sprayed at higher spray rate than the comparative formulation of example C1 (PVA+EUDRAGIT® L100-55 according to WO 2010/132204A1) without any processing problems.

**Part B: Examples 3, 4, C5 and C6: Viscosity advantage of the inventive formulation**

Procedure:

1. All ingedients were weighed accurately and mixed together in order to prepare a uniform blend.
2. The prepared blend was then added in required amount of water under overhead stirring.
3. Stirring was carried out for 60 minutes.
4. Required quantity of pH independent EUDRAGITs (NE/NM/RS/RL) were added under stirring.
5. Dispersion was filter through 60# sieve.
6. Viscosity of prepared dispersions was measured at 22 °C using Brookfield Viscometer. 500 ml glass beaker and spindle S61 was used in the study and determination was carried out at 20 RPM

**Formula**

Table 4

| Ingredients | Solids | % content | Quantity |
|---|---|---|---|
| Poval® 205S | 54.9 | 39.225493 | 54.9 |
| PEG 6000 | 16.46 | 11.760503 | 16.46 |
| Talc | 37.32 | 26.66476136 | 37.32 |
| TiO2 | 31.28 | 22.34924264 | 31.28 |
| Water | | | 419.88 |

(continued)

| Ingredients | Solids | % content | Quantity |
|---|---|---|---|
| | 139.96 | | 559.84 |
| Solid content(%w/w)= 25 | | | |

**Results:**

Table 5

| | Example 3 | Example 4 | Example C5 | Example C6 |
|---|---|---|---|---|
| **Quantity of EUDRAGIT (calculated on PVA) added into PVA dispersion** | **Viscosity(mPa.s)** | | | |
| | **EUDRAGIT® RS 30D** | **EUDRAGIT® RL 30D** | **EUDRAGIT® NM 30D** | **EUDRAGIT® NE 30D** |
| **0%** | 235.1 | 209.4 | 192 | 190.5 |
| **1%** | 189 | 117.6 | 192.3 | 187.5 |
| **2%** | 160.8 | 99.9 | 188.4 | 185.4 |
| **3%** | 142.8 | 96 | 186.6 | 177.9 |
| **4%** | 129.9 | 94.5 | 183.9 | 177.6 |
| **5%** | 120.3 | 94.2 | 180.9 | 175.5 |
| **10%** | 100 | 94 | 172.5 | 169.2 |

**Part C: Examples 7 and C8: Active Pharmaceutical Ingredient (API) advantage of the inventive formulation**

**FORMULATION DETAILS:**

**1. Preparation of Atorvastatin Drug loaded pellets:**

Formula: 10% Atorvastain coating on 400 gm Sugar spheres

| | % on API | SOLID | QTY/BATCH |
|---|---|---|---|
| Atorvastain Calcium trihydrate | | 40.00 | 40.00 |
| PVP K90 | 10.00 | 4.00 | **4.00** |
| Butylated Hydroxy Anisol (BHA) | 0.30 | 0.12 | 0.12 |
| Methanol | | | 397.08 |
| Total | | 44.12 | 441.20 |
| Table 6 / Solid content =10%w/w | | | |

Procedure:

1. PVP K 90 and BHA were dissolved in methanol under overhead stirring.

2. Atorvastatin calcium trihydrate was then added in above solution under stirring.

3. A clear solution was obtained which was used for drug loading on sugar spheres (20/25#).

4. 400 gm sugar spheres were taken in GPCG Glatt 1.1 wuster coater for drug loading.

5. The prepared solution was sprayed onto sugar spheres using following parameters:

Column Height          : 20 mm
Plate type             : B plate
Nozzle bore            : 0.8 mm
Spray rate             : 1-14 gm/min
Air flow               : 147-160 cfm
Atomization pressure   : 1-1.3 bar
Inlet temperature      : 25-26° C
Product temperature    : 24-26° C

**2. Preparation of PVA+ EUDRAGIT® RL 30D (on dry basis) Coated Atorvastatin pellets:**

**PVA+RL formulation**

Table 7

| Ingredients | % content |
|---|---|
|  |  |
| Poval® 205S | 40.29 |
| EUDRAGIT® RL 30D (on dry basis) | 0.8 |
| PEG 6000 | 12.64 |
| Talc | 25.2 |
| Titanium dioxide | 21.07 |
| water | Q.S. to prepare 15% w/w dispersion |

Procedure:

1. All ingredients were mixed together and added to required quantity of water under overhead stirring.

2. Stirring was carried out for 60 min

3. Prepared dispersion was filtered through 60# sieve. pH of dispersion was 7.10.

4. For 15% w/w solids coating on 70 gm drug loaded atorvastatin pellets, 70 gm of coating dispersion was taken.

5. Coating was carried out on Mycrolab Huttlin using following parameters:

Nozzle bore            : 0.6 mm
Spray rate             : 0.4-0.8 gm/min
Air flow               : 23-24 m$^3$/h
Atomization pressure   : 1.0 bar
Microclimate pressure  : 0.6 bar
Inlet temperature      : 34-43° C
Product temperature    : 30-38° C

**3. Preparation Opadry 200 Coated Atorvastatin pellets:**

**Opadry® 200 formulation**

Table 8

| Ingredients | % content |
|---|---|
| Opadry 200 | 100 |
| Water | Q.S. to prepare 15% w/w dispersion |

Procedure:

1. Opadry 200 was added to required quantity of water under overhead stirring.

2. Stirring was carried out for 60 min

3. Prepared dispersion was filtered through 60# sieve. pH of dispersion was 5.79.

4. For 15% w/w solids coating on 70 g drug loaded atorvastatin pellets, 70 g of coating dispersion was taken.

5. Coating was carried out on Mycrolab Huttlin using following parameters:

Nozzle bore            : 0.6 mm
Spray rate             : 0.4 gm/min
Air flow               : 23-24 m$^3$/h
Atomization pressure   : 1.0 bar
Microclimate pressure  : 0.6 bar
Inlet temperature      : 35-50° C
Product temperature    : 30-42° C

**RESULTS:**

**Analysis of Atorvastatin lactone in coated formulations:**

Procedure: For this study uncoated drug loaded pellets, Poval® 205S -

EUDRAGIT® RL 30D (on dry basis) coated pellets and Opadry® 200 coated pellets were analysed for initial Atorvastatin lactone impurity. All three pellets were also exposed to accelerated storage condition 40°C/75% RH in open petri for 45 days and generation of Atorvastatin lactone was determined. Following are the result of this study:

Table 9

| Atorvastatin pellets | Atorvastatin Lactone (%) | | Difference in impurity | % increase in lactone impurity from initial |
|---|---|---|---|---|
| | Initial | 45 days 40°C/75% RH Open petri | | |
| | | | | |
| Drug loaded pellets (Uncoated) | 0.13 | 0.32 | 0.19 | 146.15 |
| | | | | |

(continued)

| Atorvastatin pellets | Atorvastatin Lactone (%) | | Difference in impurity | % increase in lactone impurity from initial |
|---|---|---|---|---|
| | Initial | 45 days 40°C/75% RH Open petri | | |
| Poval® 205S + EUDRAGIT® RL 30D (on dry basis) coated pellets | 0.14 | 0.24 | 0.1 | 71.43 |
| | | | | |
| Opadry 200 coated pellets | 0.15 | 2.08 | 1.93 | 1286.67 |

**Example for a powder composition:**

Example-P1

| Ingredients | % content |
|---|---|
| PVA(Fine particles) | 40.29 |
| Eudragit® RL 30 D | 0.8 |
| PEG 6000 | 12.64 |
| Talc | 25.2 |
| Titanium di oxide | 21.07 |
| Total | 100 |

Example-P2

| Ingredients | % content |
|---|---|
| PVA(coarser particles) | 40.29 |
| Eudragit® RL 30 D | 0.8 |
| PEG 6000 | 12.64 |
| Talc | 25.2 |
| Titanium di oxide | 21.07 |
| Total | 100 |

1. Eudragit® RL 30 D, talc and titanium dioxide were mixed together.

2. PEG 6000 was mixed with above blend uniformly followed by PVA.

3. Blend was dried at 50° C for 30 min

Particle size analysis was carried out using USP 29 method (<786> PARTICLE SIZE DISTRIBUTION ESTIMATION BY ANALYTICAL SIEVING) by mechanical agitation.

| | Minimum pore size thru which 90% of particles pass |
|---|---|
| Example-P1 | 150 $\mu$m (US mesh #100) |

(continued)

| Example-P2 | 850 μm (US mesh #20) |
|---|---|

**Part D: Examples C9, C10, C11, 12, 13 and C14: Moisture protection of the inventive formulation at 84% relative humidity (RH)**

Table 11

| Days | Example C9 Uncoated<br>Mean % Wt gain | Example C10 Opadry® 200<br>Mean % Wt gain | Example C11 PVA+L100-55<br>Mean % Wt gain | Example 12 PVA+RL<br>Mean % Wt gain | Example 13 PVA+RS<br>Mean % Wt gain | Example C14 Opadry® AMB<br>Mean % Wt gain |
|---|---|---|---|---|---|---|
| 0 | 0 | 0 | 0 | 0 | 0 | 0 |
| 1 | 6.11 | 2.05 | 2.41 | 2.17 | 2.37 | 2.54 |
| 2 | 7.11 | 3.94 | 4.05 | 3.97 | 4.13 | 4.48 |
| 3 | 7.86 | 5.3 | 5.61 | 5.54 | 5.5 | 6.42 |
| 4 | 8.73 | 7.15 | 7.18 | 7.22 | 7.06 | 8.35 |
| 7 | 9.85 | 9.44 | 9.5 | 9.51 | 9.15 | 10.65 |

Discussion: Poval® 205S+EUDRAGIT® RS (PVA+RS) and Poval® 205S + EUDRAGIT® RL (PVA+RL) formulation gives comparable moisture protection to Opadry® 200 formulation.

**Claims**

1. Coating composition which, calculated on dry weight, is comprising at least 20 % by weight polyvinyl alcohol and 0.1 to 20 % by weight, calculated on the weight of the polyvinyl alcohol, of a water-insoluble (meth)acrylate copolymer comprising quaternary ammonium groups, wherein the viscosity of an aqueous dispersion comprising that coating composition with 25 % (w/w) solid content at 22 °C is 200 mPa·s or less measured at 22 °C using a Brookfield Viscometer.

2. Coating composition according to Claim 1, wherein further excipients add up with the polyvinyl alcohol and the water-insoluble (meth)acrylate copolymer to 100% by weight.

3. Coating composition according to Claim 2, wherein the further excipients are selected from the group of antioxidants, brighteners, binding agents, flavouring agents, flow aids, fragrances, glidants, penetration-promoting agents, pigments, plasticizers, polymers, which are not a polyvinyl alcohol or a water-insoluble (meth)acrylate copolymer, pore-forming agents or stabilizers.

4. Coating composition according to any of Claims 1 to 3, wherein the water-insoluble (meth)acrylate polymer is composed of 85 to 98% by weight of free-radical polymerized $C_1$- to $C_4$-alkyl esters of acrylic or methacrylic acid and 2 to 15 % by weight of alkyl(meth)acrylate monomers with a quaternary ammonium group in the alkyl radical.

5. Coating composition according to Claim 4, wherein the water-insoluble (meth)acrylate copolymer is composed of 55 to 75 % by weight of methyl methacrylate, 20 - 40% by weight of ethyl acrylate and 2 to 7% by weight of 2-trimethylammoniumethyl methacrylate chloride.

6. Coating composition according to Claim 4, wherein the water-insoluble (meth)acrylate copolymer is composed of 50 to 70% by weight of methyl methacrylate, 20 to 40% by weight of ethyl acrylate and more than 7 and up to 15 % by weight of 2-trimethylammoniumethyl methacrylate chloride.

7.  Coating composition according to any of Claims 1 to 6, wherein, when used for coating of atorvastatin containing pellets, the increase in impurities measured as lactone from the initial stage to 45 days storage in open petri dishes at 40 °C and 75 % relative humidity is less than 1000 %.

8.  Coating composition according to any of Claims 1 to 7, wherein a tablet coated with an aqueous dispersion comprising the coating composition disintegrates in an aqueous medium in 30 min or less.

9.  Coating composition according to any of Claims 1 to 8, which is in the form of a powder.

10.  Coating composition according to any of Claims 1 to 8, which is in the form of an aqueous dispersion.

11.  Dosage form, comprising a core, comprising an active pharmaceutical or nutraceutical ingredient and a coating derived from a coating composition according to Claims 1 to 10.

12.  Dosage form according to Claim 11, wherein the active pharmaceutical or nutraceutical ingredient is acid labile.

13.  Dosage form according to Claim 11 or 12, wherein the active ingredient is atorvastatin.

14.  Use of a coating composition according to Claims 1 to 10 for preparing a dosage form according Claims 11 to 12.

15.  Process for preparing a dosage form according to any of Claims 11 to 13 by first preparing a core comprising an active pharmaceutical or nutraceutical ingredient and subsequently applying a coating composition the form of an aqueous dispersion according to Claim 10 onto the core.


**Patentansprüche**

1.  Beschichtungszusammensetzung, die, bezogen auf das Trockengewicht, mindestens 20 Gew.-% Polyvinylalkohol und 0,1 bis 20 Gew.-%, berechnet auf das Gewicht des Polyvinylalkohols, eines wasserunlöslichen (Meth)acrylat-Copolymers mit quartären Ammoniumgruppen umfasst, wobei die bei 22 °C unter Verwendung eines Brookfield-Viskosimeters gemessene Viskosität einer wässrigen Dispersion, die die Beschichtungszusammensetzung in einem Feststoffgehalt von 25 % (w/w) umfasst, bei 22 °C 200 mPa·s oder weniger beträgt.

2.  Beschichtungszusammensetzung nach Anspruch 1, wobei weitere Hilfsstoffe sich mit dem Polyvinylalkohol und dem wasserunlöslichen (Meth)acrylat-Copolymer zu 100 Gew.-% summieren.

3.  Beschichtungszusammensetzung nach Anspruch 2, wobei die weiteren Hilfsstoffe aus der Gruppe der Antioxidantien, Aufheller, Bindemittel, Aromastoffe, Rieselhilfen, Duftstoffe, Gleitmittel, Penetrationsförderer, Pigmente, Weichmacher, Polymere, bei denen es sich nicht um einen Polyvinylalkohol oder ein wasserunlösliches (Meth)acrylat-Copolymer handelt, Porenbildner oder Stabilisatoren ausgewählt sind.

4.  Beschichtungszusammensetzung nach einem der Ansprüche 1 bis 3, wobei das wasserunlösliche (Meth)acrylat-Copolymer aus 85 bis 98 Gew.-% radikalisch polymerisierten $C_1$- bis $C_4$-Alkylestern der Acryl- oder Methacrylsäure und 2 bis 15 Gew.-% Alkyl(meth)acrylat-Monomeren mit einer quartären Ammoniumgruppe im Alkylrest besteht.

5.  Beschichtungszusammensetzung nach Anspruch 4, wobei das wasserunlösliche (Meth)acrylat-Copolymer aus 55 bis 75 Gew.-% Methylmethacrylat, 20-40 Gew.-% Ethylacrylat und 2 bis 7 Gew.-% 2-Trimethylammoniumethylmethacrylatchlorid besteht.

6.  Beschichtungszusammensetzung nach Anspruch 4, wobei das wasserunlösliche (Meth)acrylat-Copolymer aus 50 bis 70 Gew.-% Methylmethacrylat, 20-40 Gew.-% Ethylacrylat und mehr als 7 und bis zu 15 Gew.-% 2-Trimethylammoniumethylmethacrylatchlorid besteht.

7.  Beschichtungszusammensetzung nach einem der Ansprüche 1 bis 6, wobei bei Verwendung zur Beschichtung von Atorvastatin enthaltenden Pellets die Zunahme von als Lacton gemessenen Verunreinigungen vom Anfangsstadium bis 45 Tage Lagerung in offenen Petrischalen bei 40 °C und 75 % relativer Feuchte weniger als 1000 % beträgt.

8. Beschichtungszusammensetzung nach einem der Ansprüche 1 bis 7, wobei eine mit einer wässrigen Dispersion, die die Beschichtungszusammensetzung umfasst, beschichtete Tablette in einem wässrigen Medium in 30 min oder weniger zerfällt.

9. Beschichtungszusammensetzung nach einem der Ansprüche 1 bis 8, die in Form eines Pulvers vorliegt.

10. Beschichtungszusammensetzung nach einem der Ansprüche 1 bis 8, die in Form einer wässrigen Dispersion vorliegt.

11. Dosierungsform, umfassend einen Kern, der einen pharmazeutischen oder nutrazeutischen Wirkstoff umfasst, und eine Beschichtung, die sich von einer Beschichtungszusammensetzung nach den Ansprüchen 1 bis 10 ableitet.

12. Dosierungsform nach Anspruch 11, wobei der pharmazeutische oder nutrazeutischen Wirkstoff säurelabil ist.

13. Dosierungsform nach Anspruch 11 oder 12, wobei es sich bei dem Wirkstoff um Atorvastatin handelt.

14. Verwendung einer Beschichtungszusammensetzung nach den Ansprüchen 1 bis 10 zur Herstellung einer Dosierungsform nach den Ansprüchen 11 bis 12.

15. Verfahren zur Herstellung einer Dosierungsform nach den Ansprüchen 11 bis 13, bei dem man zunächst einen Kern, der einen pharmazeutischen oder nutrazeutischen Wirkstoff umfasst, herstellt und anschließend eine Beschichtungszusammensetzung in Form einer wässrigen Dispersion nach Anspruch 10 auf den Kern aufbringt.

**Revendications**

1. Composition de revêtement qui, calculé sur le poids sec, comprend au moins 20 % en poids de poly (alcool vinylique) et 0,1 à 20 % en poids, calculé sur le poids du poly(alcool vinylique), d'un copolymère insoluble dans l'eau de (méth)acrylate comprenant des groupes ammonium quaternaire, la viscosité d'une dispersion aqueuse comprenant cette composition de revêtement comportant 25 % (p/p) de teneur en solides à 22 °C étant de 200 mPa.s ou moins mesurée à 22 °C à l'aide d'un viscosimètre Brookfield.

2. Composition de revêtement selon la revendication 1, des excipients supplémentaires s'additionnant au poly(alcool vinylique) et au copolymère insoluble dans l'eau de (méth)acrylate jusqu'à 100 % en poids.

3. Composition de revêtement selon la revendication 2, les excipients supplémentaires étant choisis dans le groupe des antioxydants, des azurants, des agents liants, des agents d'arôme, des auxiliaires d'écoulement, des fragrances, des agents glissants, des agents de promotion de la pénétration, des pigments, des plastifiants, des polymères, qui ne sont pas un poly(alcool vinylique) ou un copolymère insoluble dans l'eau de (méth)acrylate, des agents porogènes ou des stabilisants.

4. Composition de revêtement selon l'une quelconque des revendications 1 à 3, le polymère insoluble dans l'eau de (méth)acrylate étant composé de 85 à 98 % en poids d'esters de $C_{1-4}$-alkyle d'acide acrylique ou méthacrylique polymérisés par radicaux libres et de 2 à 15 % en poids de monomères de (méth)acrylate d'alkyle comportant un groupe ammonium quaternaire dans le radical alkyle.

5. Composition de revêtement selon la revendication 4, le copolymère insoluble dans l'eau de (méth)acrylate étant composé de 55 à 75 % en poids de méthacrylate de méthyle, de 20 à 40 % en poids d'acrylate d'éthyle et de 2 à 7 % en poids de chlorure de méthacrylate de 2-triméthylammoniuméthyle.

6. Composition de revêtement selon la revendication 4, le copolymère insoluble dans l'eau de (méth)acrylate étant composé de 50 à 70 % en poids de méthacrylate de méthyle, de 20 à 40 % en poids d'acrylate d'éthyle et de plus de 7 et jusqu'à 15 % en poids de chlorure de méthacrylate de 2-triméthylammoniuméthyle.

7. Composition de revêtement selon l'une quelconque des revendications 1 à 6, dans laquelle, lorsqu'elle est utilisée pour le revêtement de pastilles contenant de l'atorvastatine, l'augmentation des impuretés mesurées en tant que lactone du stade initial jusqu'à 45 jours de stockage dans des boîtes de Petri ouvertes à 40 °C et 75 % d'humidité relative est inférieure à 1 000 %.

8. Composition de revêtement selon l'une quelconque des revendications 1 à 7, un comprimé revêtu avec une dispersion aqueuse comprenant la composition de revêtement se désintégrant dans un milieu aqueux en 30 min ou moins.

9. Composition de revêtement selon l'une quelconque des revendications 1 à 8, qui se trouve sous forme d'une poudre.

10. Composition de revêtement selon l'une quelconque des revendications 1 à 8, qui se trouve sous forme d'une dispersion aqueuse.

11. Forme de dosage, comprenant un noyau, comprenant un ingrédient actif pharmaceutique ou nutraceutique et un revêtement issu d'une composition de revêtement selon les revendications 1 à 10.

12. Forme de dosage selon la revendication 11, l'ingrédient actif pharmaceutique ou nutraceutique étant labile en milieu acide.

13. Forme de dosage selon la revendication 11 ou 12, l'ingrédient actif étant l'atorvastatine.

14. Utilisation d'une composition de revêtement selon les revendications 1 à 10 pour la préparation d'une forme de dosage selon les revendications 11 et 12.

15. Procédé pour la préparation d'une forme de dosage selon l'une quelconque des revendications 11 à 13 en préparant tout d'abord un noyau comprenant un ingrédient actif pharmaceutique ou nutraceutique et en appliquant subséquemment une composition de revêtement sous forme d'une dispersion aqueuse selon la revendication 10 sur le noyau.

**EP 3 033 076 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2010132204 A1